# EUROPEAN PATENT SPECIFICATION

(11) **EP 2 958 495 B1**
(45) Date of publication and mention of the grant of the patent: **18.01.2017**
(21) Application number: 13719600.2
(22) Date of filing: 19.02.2013
(51) Int. Cl.: H01R 13/629, A61B 8/00, G01S 7/52

(54) **A CONNECTION SYSTEM AND AN ULTRASOUND SYSTEM COMPRISING SAID CONNECTION SYSTEM**
VERBINDUNGSSYSTEM UND ULTRASCHALLSYSTEM MIT DIESEM VERBINDUNGSSYSTEM
SYSTÈME DE CONNEXION ET SYSTÈME ULTRASONORE COMPRENANT LEDIT SYSTÈME DE CONNEXION

(43) Date of publication of application: 30.12.2015
(73) Proprietor: Super Sonic Imagine, 13857 Aix-en-Provence Cedex (FR)
(72) Inventor: GIRAL, Frédéric, F-83470 Pourcieux (FR); MARESCHAL DE CHARENTENAY, Antoine, 01220 Divonne les Bains (FR)
(74) Representative: Cabinet Plasseraud
(86) International application number: PCT/IB2013/000421
(87) International publication number: WO 2014/128519

(56) References cited:
- WO-A1-2012/053519
- JP-A- 2008 086 653
- US-A- 5 882 310
- US-A1- 2012 078 109

## Description

### FIELD OF THE INVENTION

The present invention concerns a connection system for connecting a probe to an electronic device in an ultrasound system.

### BACKGROUND OF THE INVENTION

The present invention concerns more precisely a connection system for connecting a probe to an electronic device, wherein the probe emits and/or receives ultrasound waves into a medium, and wherein the connection system comprises:
- a first element (a receptacle) having first electrical contacts, said first element being attached to the electronic device,
- a second element (a connector) having second electrical contacts for providing an electrical connection between the first electrical contacts and the second electrical contacts, said second element being attached to the probe,
- an activation device, and
- a third element that can be moved by the activation device relative to the first element between an activated position and an inactivated position, wherein in the activated position the first electrical contacts can contact the second electrical contacts, and in the inactivated position the first electrical contacts can not contact the second electrical contacts.

It is known from the document US 5 882 310 a connection system adapted for a multiport ultrasound imaging system. The imaging system comprises a plurality of receptacles that receives a probe connector, each receptacle having a plate that is actuated by a pressure roller to rotate the receptacle contacts to the probe connector contacts.

Only one receptacle can be activated at the same time, and only one probe is connected to the ultrasound imaging system. The connection system eliminates the need of having switches inside the imaging system to select a specific ultrasound probe. Such connection system is advantageous in terms of size and cost.

However, this connection system is able connect only one connector to the imaging system at the same time. The imaging system needs to be powered to mechanically disconnect and remove a connected probe. The probe can not be easily and rapidly disconnected and removed from the electronic device.

### OBJECTS AND SUMMARY OF THE INVENTION

One object of the present invention is to provide a connection system that also eliminate the switches inside the ultrasound system, and that is improved so as it is possible to easily mechanically connect or disconnect the probe from the ultrasound system.

To this effect, the connection system is characterized in that:
- the second element can be locked to the third element by a locking mechanism so as the probe is mechanically connected to the electronic device, or the second element can be released from the third element by said locking mechanism so as the probe is mechanically disconnected from the electronic device, and
- the third element can be moved by the activation device between the activated position and the inactivated position, the second element being locked to or released from the third element.

Thanks to these features, the second element can be in a state (locked/released) independent from the position of the third element (activated/inactivated).

The probe can be removed from the ultrasound electronic device, the electrical connection between the first and second element being established or not by the actuation device.

The probe can be removed from the ultrasound electronic device rapidly by a user that manipulates the locking mechanism, without powering the electronic device. For example, the probe can be removed from the system for cleaning.

The probe is electrically connected or not to the ultrasound electronic device by the actuation device, said actuation device being powered and controlled by the electronic device.

An ultrasound electronic device having a plurality of connection systems, as defined above, for connecting a plurality of probes does not need to have switches.

The signal to noise ratio (SNR) of the received signals is also improved.

One or more than one probe can be simultaneously connected to the electronic device.

In various embodiments of the connection system, one and/or other of the following features may optionally be incorporated.

According to an aspect of the connection system, the first electrical contacts are situated according to a contacts plane and the activation device can move the third element according to an activation direction perpendicular to said contacts plane.

According to an aspect of the connection system, the second element is adapted to be inserted into or to be removed from the third element according to an insertion direction so as the locking mechanism can lock or release the second element, and wherein said insertion direction is not parallel to said contact plane.

According to an aspect of the connection system, the insertion direction is parallel to the activation direction.

According to an aspect of the connection system, the third element comprises a connecting shaft extending according said insertion direction through the first element towards the second element, so as the second element can be locked to the third element.

According to an aspect of the connection system, the actuation device is an electric linear actuator that is controlled by the electronic device.

According to an aspect of the connection system, the locking mechanism is a rotatable handle mounted on the second element, said handle being rotated by a user to lock or to release the second element onto the third element.

Another object of the present invention is to provide an ultrasound system comprising:
- a probe adapted to emit and/or to receive ultrasound waves into the medium,
- a connection system as disclosed above,
- an electronic device connected to said probe by said connection system, said electronic device comprising a processing unit, and
wherein the electronic device is adapted to control the actuation device to move the third element between the activated position and the inactivated position.

In various embodiments of the ultrasound system, one and/or other of the following features may optionally be incorporated.

According to an aspect of the ultrasound system, the actuation device and the third element are integrated with the first element inside the electronic device.

According to an aspect of the ultrasound system, it comprises a set of at least two connection systems, and the electronic device comprises:
- the first elements of said set,
- a signal bus to interconnect said first elements, and
wherein the electronic device can control each one of the activation device of the set for moving each third element of the set between the activated position and the inactivated position.

According to an aspect of the ultrasound system, it comprises a set of at least four connection systems, and the electronic device comprises:
- the first elements of said set,
- a first signal bus to interconnect a first set of two of the first elements, and
- a second signal bus to interconnect a second set of the first elements, said second set not comprising a first element of said first set.

According to an aspect of the ultrasound system, the connection systems are organized according to a matrix arrangement.

According to an aspect of the ultrasound system, it comprises a connection unit comprising:
- a first element belonging to the first set and a first element belonging to the second set,
- a single activation device, and
- a single third element moved by said activation device and adapted to receive and lock a second element from a first probe for establishing electrical contacts between said second element and the first element belonging to the first set, and adapted to receive and lock a second element from a second probe for establishing electrical contacts between said a second element and the first element belonging to the second set.

### BRIEF DESCRIPTION OF THE DRAWINGS

Other features and advantages of the invention will be apparent from the following detailed description of at least one of its embodiments given by way of non-limiting example, with reference to the accompanying drawings. In the drawings:
- Figure 1 is schematic view of an imaging system comprising a probe, an electronic device and a connection system according to the invention;
- Figure 2 is a view of an electronic device for connecting a plurality of probes;
- Figure 3 is a schematic view of the electronic device of figure 2;
- Figure 4 is a schematic view of an electronic device for connecting simultaneously a plurality of probes;
- Figure 5 is a perspective view of a connection system according to a first embodiment of the invention;
- Figure 6 is a perspective cut of figure 5;
- Figure 7 is a perspective view of a connection system according to a second embodiment of the invention.

### MORE DETAILLED DESCRIPTION

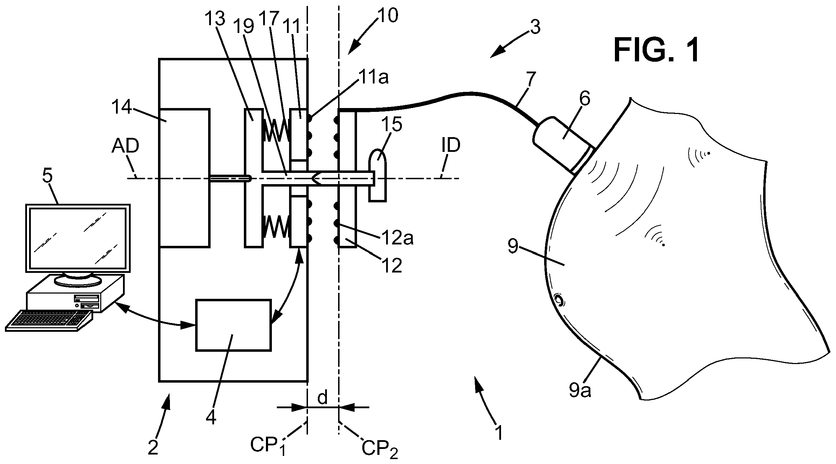

The **figure 1** is a schematic view showing an ultrasound system 1 comprising an electronic device 2 and a probe 3 that are connected one to the other by a connection system 10 corresponding to a first embodiment of the invention. The ultrasound system 1 may be an imaging ultrasound system that builds an image of a medium on the basis of received ultrasound waves, or a treatment ultrasound system that modifies the medium at least at a predetermined focal point inside the medium, or both or them doing imaging and treatment. It is understood that the treatment can be applied to non organic or organic material. In case of organic material, it could be applied to in vitro applications or to in vivo application.

**The electronic device 2** sends excitation signals to the probe 3 via the connection system 10 so as to emit at least an ultrasound wave into a medium 9. The scattering of the wave inside the medium generates backwards reflected ultrasound waves that are sensed by the probe 3 and returned in the form of reflection signals to the electronic device 2 via the connection system 10. Such method applies to ultrasound imaging of the medium as it is well known. The following description will mainly explained in view of imaging application. However, it may be also applied to the treatment application.

The electronic device 2 comprises:
- a first element 11 belonging to the connection system 10 for sending to the probe 3 said excitation signals and for receiving from the probe 3 the reflection signals,
- a processing unit 4 to control said probe 3, for example for controlling the excitation signals and for processing the received signals to compute an image representing the medium 9, and
- a screen 5 to visualize the image to a user.

The probe 3 comprises:
- a second element 12 belonging to the connection system 10, said second element 12 being adapted to cooperate with the first element 11 to electrically connect the probe 3 to the electronic device 2,
- a head 6 comprising a plurality of transducer elements, e.g. piezoelectric elements, each of them transforming the electrical signals (excitation and reflection signals) into an ultrasound wave or inversely, and
- a cable 7 that links the connection system 10 to the head 6.

The cable 7 is flexible. A user can move the head 6 above an external surface 9a of the medium 9 to get a plurality of images from the ultrasound system, each of said images representing a slice of the inner densities of the medium 9 at the plurality of positions of the head 6 above the external surface 9a.

Therefore, **the connection system 10** comprises at least:
- a first element 11 having first electrical contacts 11a, said first element 11 being attached to the electronic device 2, and
- a second element 12 having second electrical contacts 12a for providing an electrical connection between the first electrical contacts 11a and the second electrical contacts 12a, said second element 12 being attached to the probe 3.

When the first and second electrical contacts 11a, 12a are into contact to each other, the first and second electrical contacts transmit electrical signals from the probe 3 to the electronic device 2 and from the electronic device 2 to the probe 3.

The connection system 10 further comprises:
- an actuation device 14, and
- a third element 13.

The actuation device 14 may be any type of actuator that is able to move the third element 13 relative to the first element 11 between an activated position and an inactivated position. The actuation device 14 may be an electric linear actuator, an electric rotation actuator, an electric motor, an electric step motor, an electromagnet.

The third element 13 may be suspended relative to the electronic device 2 or first element 11 by an elastic element 17, e.g. a coil spring. The position of the third element 13 without actuation device action can be predetermined.

The actuation device 14 and the third element 13 are preferably integrated inside the electronic device 2 as disclosed in the herein presented embodiments. The electronic device 2 directly controls and powers the actuation device 14, and the second element 12 (probe connector) is compact in size and light. The second element 12 belonging to the probe 3 is therefore not modified compared to the known elements of already available probes. The connection device 10 according to the herein disclosed embodiments allows backward compatibility: Existing probes 3 can be used on an electronic device 2 incorporating said connection systems 10.

The actuation device 14 may be alternatively integrated inside the probe 3.

The second element 12 is linked to the third element 13 as explained in the following description.

In the activated position of the third element 13, the first element 11 is proximal to the second element 12 so as the first electrical contacts 11a can contact the second electrical contacts 12a to established electric continuity.

In the inactivated position of the third element 13, the first element 11 is distant from the second element 12 so as first electrical contacts 11a can not contact the second electrical contacts 12a. For example, the first electrical contacts 11a are at a distance from the second electrical contacts 12a that is higher than 0.5 mm. The above distance is for example a distance between a first contacts plane CP₁ defined by the first electrical contacts 11a and a second contact plane CP₂ defined by the second electrical contacts 12a, in said inactivated position of the third element 13.

The second element 12 can be removably fixed or locked to the third element 13 by a locking mechanism 15 to mechanically connect, plug, or lock the probe 3 to the electronic device 2. The second element 12 is then in a locked state.

The second element 12 can be released from the third element 13 by the same locking mechanism 15 to mechanically disconnect, unplug or release the probe 3 from the electronic device 2. The second element 12 is then in a released state.

The third element 13 is an element that is intermediate between the first and second elements 11, 12 of the connection system 10. Thanks to this third element 13, the second element 12 from the probe can be mechanically locked on this third element 13 by the locking mechanism 15. Thanks to the actuation device 14, the third element 13 can be moved relative to the first element 11 for electrically connecting or not the second element 12 to the first element 11.

Contrary to prior art connection systems, the connection system 10 of present invention can provide an electrical connection of the probe 3 that is independent from the mechanical locking of the probe 3 to the electronic device 2.

As soon as the probe 3 is removed from the electronic device 2, after being mechanically disconnected from it, the probe 3 is also electrically disconnected from the electronic device 2: The first and second electrical contacts 11a, 12a do not contact each other and the electrical continuity is broken between the electronic device 2 and the probe 3.

The third element 13 of the connection system 10 can be moved by the activation device 14 from the activated position to the inactivated position, or from the inactivated position to the activated position, the second element 12 of the connection system 10 being in any state: the locked state or the released state, i.e. the second element 12 being locked to the third element 13 or being released from the third element 13.

Therefore, the connection system 10 can be in any of the four following working states S1, S2, S3, S4, that correspond to all the possibilities of the third element 13 positions and the second element 12 states.

The second element 12 is switched between the released state and the locked state by the locking mechanism 15. The third element 13 is switched between the inactivated position and the activated position by the activation device 14.

**Table 1: all the working states of the connection system according to the invention**

| | | Third element (13) positions | |
|---|---|---|---|
| | | Inactivated position | Activated position |
| Second element (12) states | Released | S1 | S3 |
| | Locked | S2 | S4 |

In the working state S1, the third element 13 is in the inactivated position. The first and second electrical contacts 11a, 12a are not electrically connected to each other. The second element 12 is in the released state. The second element 12 can be removed from the third element 13. The probe 3 can be mechanically removed from the electronic device 2. The locking mechanism 15 may be activated by a user to switch from the working state S1 to the working state S2, to lock a probe 3 on the electronic device 2. Additionally, if no probe is present, the processing unit 4 may switch the third element 13 from the inactivated position (working state S1) to the activated position (working state S3). In that case, the third element 13 is moved by the activation device 14 without any probe.

In the working state S2, the second element 12 is now locked on the third element 13, and the probe 3 is locked on the electronic device 2 and can not be directly removed from the third element 13. The locking mechanism 15 must be activated by a user to switch back to the working state S1 and to release the second element 12, i.e. probe 3. Alternatively, the user may interfere with the ultrasound imaging system 1 and the electronic device 2 (processing unit 4) may switch the third element 13 from the inactivated position to the activated position to switch from the working state S2 to the working state S4.

In the working state S3, the third element 13 is in the activated position. The second element 12 is in the released state, and can be removed from the third element 13. The probe 3 can be mechanically removed from the electronic device 2. The locking mechanism 15 may be activated by a user to switch from the working state S3 to the working state S4 to lock the probe 3 on the electronic device 2. Such working state S3 is therefore a state that should be transient, and that should not be a normal use state.

In the working state S4, the second element 12 is locked on the third element 12, and the probe 3 is also locked on the electronic device 2. The locking mechanism 15 must be activated by a user to switch back to the working state S3 (unlocking or releasing of the probe). The processing unit 4 may switch the third element 13 from the activated position to the inactivated position to switch from the working state S4 to the working state S2.

The switch between the working states S2 and S4 correspond to the activation of connection system 10. This electrically connects or not the first electrical contacts 11a with the second electrical contacts 12a. The processing unit 4 can switch the third element 13 from the inactivated position (working state S2) to the activated position (working state S4). In that case, the third element 13 is moved by the activation device 14, the second element 12 of the probe 3 being installed inside the connection system 10. The actuation device 14 is therefore moving (controlling) the first and second electrical contacts 11A, 12a so as they are switch to electrical contacts or not. They are functioning like electrical switches that are usually used for connecting only one particular selected probe in an electronic device 2 having a plurality of probes connected on it.

Thanks to the above four working states, the second element 12 can be locked or released from the third element 13 independently from the position of the third element 13 relative to the first element 11: in the activated or inactivated position. The probe 3 connected to the connection system 10 can be removed from the ultrasound electronic device 2 rapidly by a user.

The activation device 14 is preferably moving the third element 13 according to an activation direction AD that is perpendicular to the first contact plane CP₁. The first and second contact planes CP₁, CP₂ are preferably parallel to each other.

The second element 12 is for example locked or released to the third element 13 according to an insertion direction ID, said insertion direction being not parallel to the first contact plane CP₁, and being preferably perpendicular to said first contact plane CP₁. The second element 12 is therefore locked or released to the third element according to a direction that is the same as the activation direction AD that moves the third element 13 relative to the first element 11.

The connection system may have the following features: The third element 13 is situated inside the electronic device bellow the first element 11 so as it is directly moved and controlled by the actuation device 14 inside the electronic device 2. The first element 11 is situated at an external surface of a casing of the electronic device so as to cooperate with the second element 12 from the probe 3. The first element 11 comprises a hole, and the third element 13 comprise a connecting shaft 19 extending at least partially through said hole to the outside of the electronic device 2. The connecting shaft 19 can therefore cooperate with the locking mechanism 15 of the probe 3 so as to lock or unlock the second element 12 to the third element 13: The second element 12 is at positioned at a predetermined position relative to the third element 13. The shaft extends according to the insertion direction ID through the hole of the first element 11.

The locking mechanism 15 is capable to fix or lock the second element 12 at said predetermined position (locked state) or to release the second element 12 so as it can be removed from the electronic device 2. The locking mechanism comprises (at a first end in the direction of the electronic device) a nose adapted to cooperate with the connecting shaft 19 for locking or releasing said second element 12.

The locking mechanism 15 may be of any kind. For example, it is a rotatable handle mounted on the second element 12, said handle being rotated by a user to lock or release the second element from the third element 13. The probe 3 can therefore be extracted from the electronic device 2 at any time: The third element 13 being in an activated or inactivated position, and the electronic device 2 being powered or not.

**The electronic device 2** may advantageously comprise a plurality of first elements 11 for connecting a plurality of probes. Usually, such electronic device 2 comprises inner electrical switches to select simultaneously only one probe. The patent US 4 726 230 discloses an example of such electronic device.

The actuation device 14 in present invention replaces the electrical switches included inside the above prior art imaging system. In case of a connection system having lots of electrical contacts (for probes having lots of transducers elements), this is really advantageous: For example, in case of a probe head 6 having a number of 500 transducers elements, such prior art electronic device must incorporate the same number of electrical switches for each one connection system, i.e. for each probe slot (receptacle) of the electronic device. Such architecture leads to an enormous number of electrical switches that use electronic board area and that are costly.

By using the connection system 10 as a switch for all the electrical contacts or wires from a probe, the need for all these switches is eliminated. The document US 5 882 310 discloses an electronic device that comprises such controlled connection system, but this system permits to electrically connect simultaneously only one probe to the electronic device 2. When electrically connected, such connection system is also mechanically locked, and the probe can not be easily unplugged from the electronic device. Such connection system can only work in the above defined working states S1 and S4, and can not work in the working states S2 and S3.

The connection system 10 according to the invention therefore differs in that the electrical connection of the probe 3 to the electronic device 2 is independent from the mechanical locking of the probe 3 to said electronic device 2.

The actuation device 14 of present invention may use some space inside the electronic device 2, but an electronic device 2 incorporating said connection system 10 can have a reduced size. And, it is also less costly.

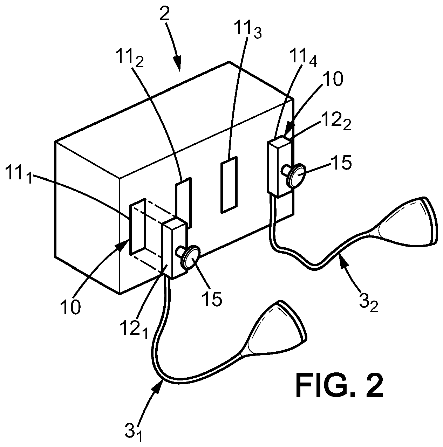

**Figure 2** shows a first example of an electronic device 2 used in conjunction with a plurality of connection systems 10. In the figure, the electronic device 2 comprises four first elements. The first elements 11 are individually denoted with the reference numbers 11₁, 11₂, 11₃, and 11₄.

A first probe 3₁ comprises a second element 12₁ and is ready to be connected to the first element 11₁. A second probe 3₂ comprising a second element 12₂ is already connected to the first element 11₄. The first elements 11₂ and 11₃ are not presently used. The user mechanically plugs or unplugs each of the probes 3₁, 3₂ to the electronic device 2 by their locking mechanisms 15.

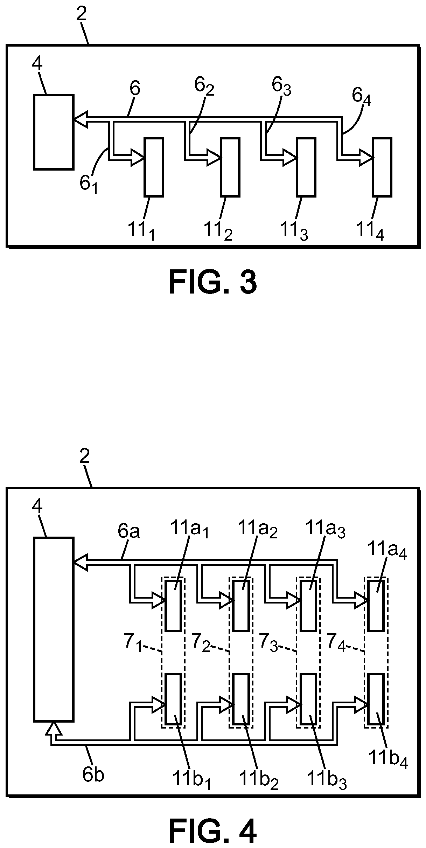

As illustrated in **figure 3****,** the electronic device 2 comprises a processing unit 4 and first elements 11₁ to 11₄. All the first electrical contacts of the first elements are connected to the processing unit 4 via a signal bus 6 comprising for example a number of lines equal to the number of first electrical contacts of one of the first elements. The signal bus 6 comprises a plurality of branches (6₁, 6₂, 6₃, 6₄), one branch for connecting one of the first elements (11₁, 11₂, 11₃, 11₄). An electrical contact of a first element 11 is therefore connected to an equivalent electrical contact of another first element 11.

**Figure 4** shows a second example of an electronic device 2 used in conjunction with a plurality of connection systems 10.

The electronic device 2 comprises first elements organized according to a matrix arrangement, having rows and columns. The electronic device 2 also comprises a number of signal buses corresponding to the number of rows to interconnect the first elements belonging to said row.

Thanks to the above feature, a plurality of probes may be connected simultaneously, one on each row. The number of transducer elements can therefore be increased. By using two probes having for example 128 transducers elements, the imaging system can behave such as having a unique 256 transducer elements. Different probes can be also connected simultaneously: probes having different transducer elements arrangements (linear, curved, matrix), or probes for different use (a probe for imaging the medium, or a probe for medium treatment). Each signal bus may comprise electronic interface adapted to the use of the probes that can be connected to it (imaging or treatment). The electronic interface for example comprises amplifies, multiplexers, analog to digital converters, digital to analog converters, etc... as it is well known.

For example, the electronic device of **figure 4** comprises two rows and four columns. The first row comprises a fist signal bus 6a to interconnect the first elements, denoted with the reference numbers 11a₁, 11a₂, 11a₃, and 11a₄, via fist branches of said first bus 6a. Similarly, the second row comprises a second signal bus 6b to interconnect the first elements denoted with the reference numbers 11b₁, 11b₂, 11b₃, and 11b₄ via second branches of said second bus 6b. The first and second signal buses 6a, 6b are linked to the processing unit 4 separately.

According to a first variant, all the first elements 11 of the matrix arrangement may be independent from the other. Each first element 11 is for example embedded inside a connection unit comprising said first element 11, a single activation device 14, and a single third element 13 actuated by said activation device 14.

According to a second variant, the first elements 11 of a column (of index i) may be grouped into a connection unit that comprises the first elements 11a₁, 11bᵢ, a single activation device 14, and a single third element 13 actuated by said activation device 14. However one or two distinct probes can be connected to the single third element 13 of said connection unit. The probes are therefore independently mechanically plugged or unplugged to the connection unit (electronic device 2), but the single actuation device 14 electrically connect or the probes by moving said single third element 13 between the inactivated position and the activated position.

Such electronic device 2 can provide numerous configurations. For example, a first probe can be plugged to the first row of a column, and a second probe can be plugged to the second row of another column. In that case, both probes are independently controlled.

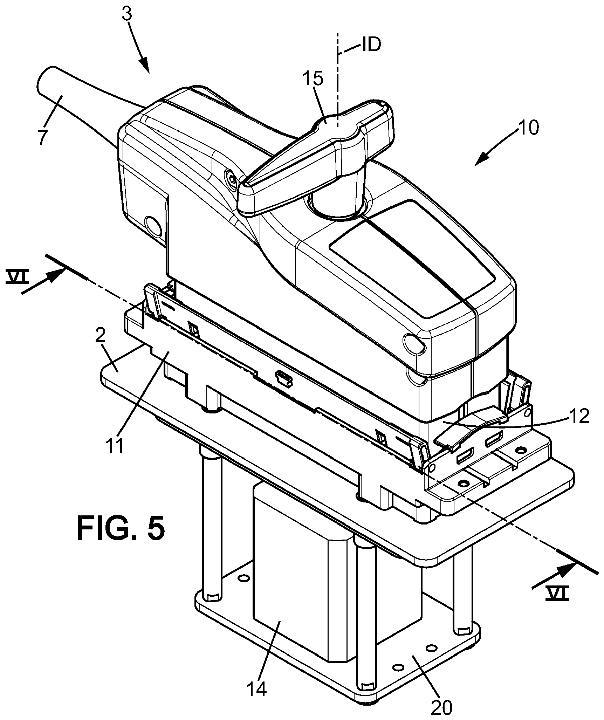

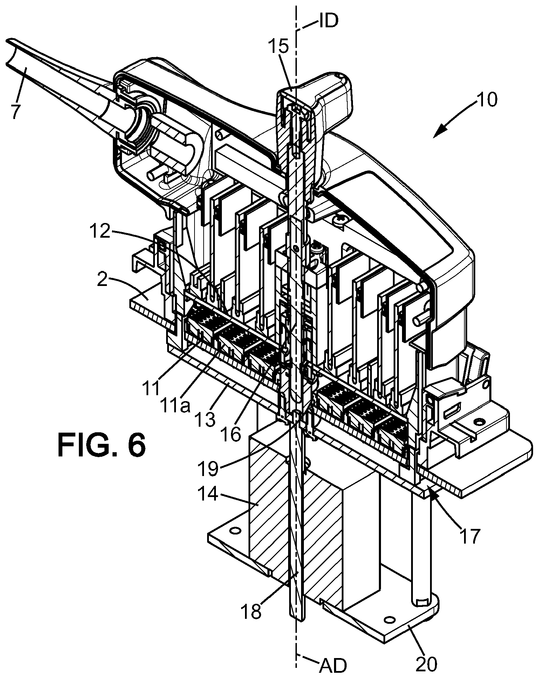

**Figures 5** **and** **6** are representing a first embodiment of the connection system 10 wherein the electronic device 2 comprises connection unit incorporating a first element 11, a third element 13 and an activation device 14. The activation device 14 is a linear motor actuating a shaft according to the activation direction AD. The activation device 14 is also mounted on a mounting plate 20 fixed inside the electronic device 2. The shaft can move the third element 13 according said acttivation direction AD.

The probe 3 comprises a locking mechanism 15 in the form of a manual handle that is rotatable according to the insertion direction ID, said insertion direction being parallel to the activation direction AD. The locking mechanism comprises a locking shaft 16 adapted to be locked to the third element 13, via the connecting shaft 19.

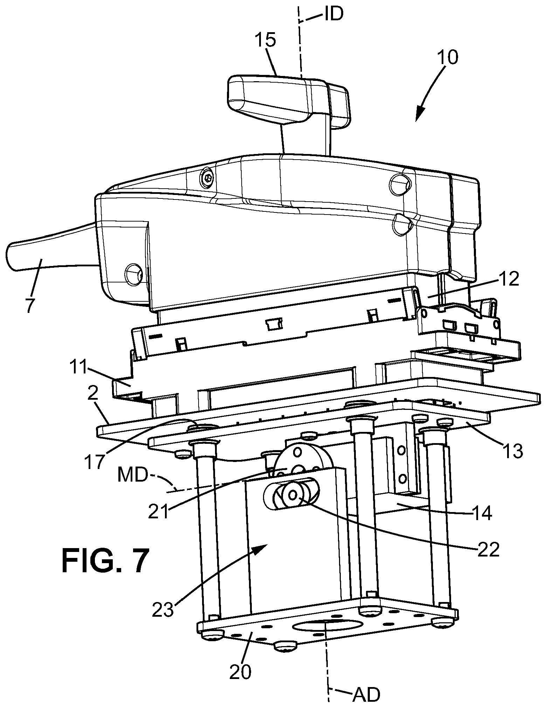

**Figure 7** represents a second embodiment of the connection system 10 wherein the actuation device 14 is a rotation motor. Said rotation motor rotates a wheel 21 having an eccentric pin. The eccentric pin is engaged inside an elongated hole of a support 23 fixed to the mounting plate 20. The rotation movement of the actuation device 14 is transformed into a linear displacement of the third element 13.

## Claims

1. **A connection system (10)** for connecting a probe to an electronic device, wherein the probe emits and/or receives ultrasound waves into a medium, and wherein the connection system comprises:
- a first element (11) having first electrical contacts, said first element configured to be attached to the electronic device,
- a second element (12) having second electrical contacts for providing an electrical connection between the first electrical contacts and the second electrical contacts, said second element configured to be attached to the probe,
- an activation device (14), and
- a third element (13) that can be moved by the activation device (14) relative to the first element (11) between an activated position and an inactivated position, wherein in the activated position the first electrical contacts can contact the second electrical contacts, and in the inactivated position the first electrical contacts can not contact the second electrical contacts,
said connection system **being characterized in that:**
- the second element (12) can be locked to the third element (13) by a locking mechanism (15) so that the probe is mechanically connected to the electronic device, or the second element (12) can be released from the third element (13) by said locking mechanism (15) so that the probe is mechanically disconnected from the electronic device, and
- the third element (13) can be moved by the activation device (14) between the activated position and the inactivated position, the second element (12) being locked to or released from the third element (13).

2. The connection system according to claim 1, wherein the first electrical contacts (11a) are situated according to a contacts plane (CP₁) and the activation device (14) can move the third element (13) according to an activation direction (AD) perpendicular to said contacts plane (CP).

3. The connection system according to claim 2, wherein the second element (12) is adapted to be inserted into or to be removed from the third element (13) according to an insertion direction (ID) so as the locking mechanism (15) can lock or release the second element (12), and wherein said insertion direction (ID) is not parallel to said contact plane (CP).

4. The connection system according to claim 3, wherein the insertion direction (ID) is parallel to the activation direction (AD).

5. The connection system according to claim 3, wherein the third element (13) comprises a connecting shaft (19) extending according said insertion direction (ID) through the first element (11) towards the second element (12), so as the second element (12) can be locked to the third element (13).

6. The connection system according to any one of the claims 1 to 5, wherein the actuation device (14) is an electric linear actuator that is controlled by the electronic device.

7. The connection system according to any one of the claims 1 to 6, wherein the locking mechanism (15) is a rotatable handle mounted on the second element (12), said handle being rotated by a user to lock or to release the second element (12) onto the third element (13).

8. **An ultrasound system (1)** comprising:
- a probe (3) adapted to emit and/or to receive ultrasound waves into the medium,
- a connection system (10) according to any one of the claims 1 to 7,
- an electronic device (2) connected to said probe (3) by said connection system (10), said electronic device comprising a processing unit (4), and
**wherein** the electronic device (2) is adapted to control the actuation device (14) to move the third element (13) between the activated position and the inactivated position.

9. The ultrasound system according to claim 8, wherein the actuation device (14) and the third element (13) are integrated with the first element (11) inside the electronic device (2).

10. The ultrasound system according to claim 8 comprising a set of at least two connection systems (10) according to any one of the claims 1 to 7, wherein the electronic device (2) comprises:
- the first elements (11) of said set,
- a signal bus (6) to interconnect said first elements (11), and
wherein the electronic device (2) can control each one of the activation device (14) of the set for moving each third element (13) of the set between the activated position and the inactivated position.

11. The ultrasound system according to claim 8 comprising a set of at least four connection systems (10) according to any one of the claims 1 to 7, wherein the electronic device (2) comprises:
- the first elements (11) of said set,
- a first signal bus (6a) to interconnect a first set of two of the first elements (11), and
- a second signal bus (6b) to interconnect a second set of the first elements (11), said second set not comprising a first element of said first set.

12. The ultrasound system according to claim 11, wherein the connection systems (10) are organized according to a matrix arrangement.

13. The ultrasound system according to claim 11 comprising a connection unit comprising:
- a first element (11a₁) belonging to the first set and a first element (11b₁) belonging to the second set,
- a single activation device (14), and
- a single third element (13) moved by said activation device and adapted to receive and lock a second element (12₁) from a first probe (3₁) for establishing electrical contacts between said second element (12₁) and the first element (11a₁) belonging to the first set, and adapted to receive and lock a second element (12₂) from a second probe (3₂) for establishing electrical contacts between said a second element (12₂) and the first element (11b₁) belonging to the second set.

## Patentansprüche

1. **Verbindungssystem (10)** zum Verbinden einer Sonde mit einer elektronischen Vorrichtung, wobei die Sonde Ultraschallwellen in ein Medium sendet und/oder empfängt, und wobei das Verbindungssystem Folgendes umfasst:
- ein erstes Element (11), das erste elektrische Kontakte hat, wobei das erste Element konfiguriert ist, um an der elektronischen Vorrichtung angebracht zu werden,
- ein zweites Element (12), das zweite elektrische Kontakte hat, um eine elektrische Verbindung zwischen den ersten elektrischen Kontakten und den zweiten elektrischen Kontakten bereitzustellen, wobei das zweite Element konfiguriert ist, um an der Sonde angebracht zu werden,
- eine Aktivierungsvorrichtung (14), und
- ein drittes Element (13), das von der Aktivierungsvorrichtung (14) in Bezug zu dem ersten Element (11) zwischen einer aktivierten Position und einer nicht aktivierten Position bewegt werden kann, wobei die ersten elektrischen Kontakte in der aktivierten Position die zweiten elektrischen Kontakte berühren können, und wobei die ersten elektrischen Kontakte in der nicht aktivierten Position die zweiten elektrischen Kontakte nicht berühren können,
wobei das Verbindungssystem **dadurch gekennzeichnet ist, dass**:
- das zweite Element (12) an dem dritten Element (13) durch einen Verriegelungsmechanismus (15) derart verriegelt werden kann, dass die Sonde mechanisch mit der elektronischen Vorrichtung verbunden ist, oder das zweite Element (12) von dem dritten Element (13) durch den Verriegelungsmechanismus (15) derart freigegeben werden kann, dass die Sonde mechanisch von der elektronischen Vorrichtung getrennt ist, und
- das dritte Element (13) von der Aktivierungsvorrichtung (14) zwischen der aktivierten Position und der nicht aktivierten Position bewegt werden kann, wobei das zweite Element (12) an dem dritten Element (13) verriegelt oder von ihm freigegeben ist.

2. Verbindungssystem nach Anspruch 1, wobei die ersten elektrischen Kontakte (11a) gemäß einer Kontaktebene (CP₁) liegen können, und die Aktivierungsvorrichtung (14) das dritte Element (13) gemäß einer Aktivierungsrichtung (AD) senkrecht zu der Kontaktebene (CP) bewegen kann.

3. Verbindungssystem nach Anspruch 2, wobei das zweite Element (12) angepasst ist, um in das dritte Element (13) gemäß einer Einführrichtung (ID) eingeführt oder von ihm entfernt zu werden, so dass der Verriegelungsmechanismus (15) das zweite Element (12) verriegeln oder freigeben kann, und wobei die Einführrichtung (ID) zu der Kontaktebene (CP) nicht parallel ist.

4. Verbindungssystem nach Anspruch 3, wobei die Einfügerichtung (ID) zu der Aktivierungsrichtung (AD) parallel ist.

5. Verbindungssystem nach Anspruch 3, wobei das dritte Element (13) einen Verbindungsschaft (19) umfasst, der sich gemäß der Einführrichtung (ID) durch das erste Element (11) zu dem zweiten Element (12) derart erstreckt, dass das zweite Element (12) an dem dritten Element (13) verriegelt werden kann.

6. Verbindungssystem nach einem der Ansprüche 1 bis 5, wobei die Betätigungsvorrichtung (14) ein elektrischer linearer Aktuator ist, der von der elektronischen Vorrichtung gesteuert wird.

7. Verbindungssystem nach einem der Ansprüche 1 bis 6, wobei der Verriegelungsmechanismus (15) ein drehbarer Griff ist, der auf das zweite Element (12) montiert ist, wobei der Griff von einem Benutzer gedreht werden kann, um das zweite Element (12) an dem dritten Element (13) zu verriegeln oder von ihm freizugeben.

8. **Ultraschallsystem (1),** das Folgendes umfasst:
- eine Sonde (3), die angepasst ist, um Ultraschallwellen in ein Medium zu senden und/oder zu empfangen,
- ein Verbindungssystem (10) nach einem der Ansprüche 1 bis 7,
- eine elektronische Vorrichtung (2), die mit der Sonde (3) durch das Verbindungssystem (10) verbunden ist, wobei die elektronische Vorrichtung eine Verarbeitungseinheit (4) umfasst, und
wobei die elektronische Vorrichtung (2) angepasst ist, um die Betätigungsvorrichtung (14) zu steuern, um das dritte Element (13) zwischen der aktivierten Position und der nicht aktivierten Position zu bewegen.

9. Ultraschallsystem nach Anspruch 8, wobei die Betätigungsvorrichtung (14) und das dritte Element (13) mit dem ersten Element (11) innerhalb der elektronischen Vorrichtung (2) integriert sind.

10. Ultraschallsystem nach Anspruch 8, das einen Satz aus mindestens zwei Verbindungssystemen (10) nach einem der Ansprüche 1 bis 7 umfasst, wobei die elektronische Vorrichtung (2) Folgendes umfasst:
- die ersten Elemente (11) des Satzes,
- einen Signalbus (6) zum Verschalten mit den ersten Elementen (11), und
wobei die elektronische Vorrichtung (2) jede der Aktivierungsvorrichtungen (14) des Satzes zum Bewegen jedes dritten Elements (13) des Satzes zwischen der aktivierten Position und der nicht aktivierten Position steuern kann.

11. Ultraschallsystem nach Anspruch 8, das einen Satz aus mindestens vier Verbindungssystemen (10) nach einem der Ansprüche 1 bis 7 umfasst, wobei die elektronische Vorrichtung (2) Folgendes umfasst:
- erste Elemente (11) des Satzes,
- einen ersten Signalbus (6a) zum Verschalten eines ersten Satzes aus den zwei ersten Elementen (11), und
- einen zweiten Signalbus (6b) zum Verschalten eines zweiten Satzes der ersten Elemente (11), wobei der zweite Satz kein Element des ersten Satzes umfasst.

12. Ultraschallsystem nach Anspruch 11, wobei die Verbindungssysteme (10) gemäß einer Matrixanordnung organisiert sind.

13. Ultraschallsystem nach Anspruch 11, das eine Verbindungseinheit umfasst, die Folgendes umfasst:
- ein erstes Element (11a₁), das zu dem ersten Satz gehört, und ein erstes Element (11b₁), das zu dem zweiten Satz gehört,
- eine einzige Aktivierungsvorrichtung (14), und
- ein einziges drittes Element (13), das von der Aktivierungsvorrichtung bewegt wird und angepasst ist, um ein zweites Element (12₁) von einer ersten Sonde (3₁) aufzunehmen und zu verriegeln, um elektrische Kontakte zwischen dem zweiten Element (12₁) und dem ersten Element (11a₁), das zu dem ersten Satz gehört, herzustellen, und angepasst ist, um ein zweites Element (12₂) von einer zweiten Sonde (3₂) zu empfangen und verriegeln, um elektrische Kontakte zwischen dem einen zweiten Element (12₂) und dem ersten Element (11b₁), das zu dem zweiten Satz gehört, herzustellen.

## Revendications

1. **Système de connexion (10)** pour connecter une sonde à un dispositif électronique, dans lequel la sonde émet et/ou reçoit des ondes ultrasonores dans un milieu, et dans lequel le système de connexion comprend :
- un premier membre (11) doté de premiers contacts électriques, ledit premier membre configuré pour être raccordé au dispositif électronique,
- un deuxième membre (12) doté de deuxièmes contacts électriques pour fournir une connexion électrique entre les premiers contacts électriques et les deuxièmes contacts électriques, ledit deuxième membre configuré pour être raccordé à la sonde,
- un dispositif d'activation (14), et
- un troisième membre (13) pouvant être déplacé par le dispositif d'activation (14) par rapport au premier élément (11) entre une position activée et une position inactivée, dans lequel en position activée les premiers contacts électriques peuvent être en contact avec les deuxièmes contacts électriques, et en position inactivée les premiers contacts électriques ne peuvent pas être en contact avec les deuxièmes contacts électriques, ledit système de connexion **étant caractérisé en ce que :**
- le deuxième membre (12) peut être verrouillé sur le troisième élément (13) par un mécanisme de verrouillage (15) de sorte que la sonde soit mécaniquement connectée au dispositif électronique, ou que le deuxième membre (12) puisse être relâché du troisième membre (13) par ledit mécanisme de verrouillage (15) de sorte que la sonde soit mécaniquement déconnectée du dispositif électronique, et
- le troisième membre (13) peut être déplacé par le dispositif d'activation (14) entre la position activée et la position inactivée, le deuxième membre (12) étant verrouillé sur ou relâché du troisième membre (13).

2. Système de connexion selon la revendication 1, dans lequel les premiers contacts électriques (11a) sont disposés selon un plan de contact (CP₁) et le dispositif d'activation (14) peut déplacer le troisième membre (13) selon une direction d'activation (AD) perpendiculaire audit plan de contact (CP).

3. Système de connexion selon la revendication 2, dans lequel le deuxième membre (12) est adapté pour être inséré dans ou retiré du troisième membre (13) selon une direction d'insertion (ID) de sorte que le mécanisme de verrouillage (15) puisse verrouiller ou relâché le deuxième membre (12), et dans lequel ladite direction d'insertion (ID) n'est pas parallèle audit plan de contact (CP).

4. Système de connexion selon la revendication 3, dans lequel la direction d'insertion (ID) et parallèle à la direction d'activation (AD).

5. Système de connexion selon la revendication 3, dans lequel le troisième membre (13) comprend un arbre de connexion (19) s'étendant selon ladite direction d'insertion (ID) à travers le premier membre (11) vers le deuxième membre (12), de sorte que le deuxième membre (12) puisse être verrouillé sur le troisième membre (13).

6. Système de connexion selon l'une quelconque des revendications 1 à 5, dans lequel le dispositif d'actionnement (14) est un vérin électrique linéaire contrôlé par le dispositif électronique.

7. Système de connexion selon l'une quelconque des revendications 1 à 6, dans lequel le mécanisme de verrouillage (15) est une poignée rotative fixée sur le deuxième membre (12), ladite poignée étant mise en rotation par un utilisateur pour verrouiller ou relâcher le deuxième membre (12) sur le troisième membre (13).

8. **Système ultrasonore (1)** comprenant :
- une sonde (13) adaptée pour émettre et où recevoir des ondes ultrasonores dans le milieu,
- un système de connexion (10) selon l'une quelconque des revendications 1 à 7,
- un dispositif électronique (2) connecté à ladite sonde (3) par ledit système de connexion (10), ledit dispositif électronique comprenant une unité de traitement (4), et
**dans lequel** le dispositif électronique (2) est adapté pour contrôler le dispositif d'actionnement (14) pour déplacer le troisième membre (13) entre la position activée et la position inactivée.

9. Système ultrasonore selon la revendication 8, dans lequel le dispositif d'actionnement (14) et le troisième élément (13) sont intégrés au premier élément (11) à l'intérieur du dispositif électronique (2).

10. Système ultrasonore selon la revendication 8 comprenant un ensemble d'au moins deux systèmes de connexion (10) selon l'une quelconque des revendications 1 à 7, dans lequel le dispositif électronique (2) comprend :
- les premiers membres (11) dudit ensemble,
- un bus de signal (6) pour interconnecter lesdits premiers membres (11), et
dans lequel le dispositif électronique (2) peut contrôler chaque dispositif d'activation (14) de l'ensemble pour déplacer chaque troisième membre (13) de l'ensemble entre la position activée et la position inactivée.

11. Système ultrasonore selon la revendication 8 comprenant un ensemble d'au moins quatre systèmes de connexion (10) selon l'une quelconque des revendications 1 à 7, dans lequel le dispositif électronique (2) comprend :
- les premiers membres (11) dudit ensemble,
- un premier bus de signal (6a) pour interconnecter un premier ensemble de deux des premiers membres (11), et
- un deuxième bus de signal (6b) pour interconnecter un deuxième ensemble des premiers membres (11), ledit deuxième ensemble ne comprenant pas un premier membre dudit premier ensemble.

12. Système ultrasonore selon la revendication 11, dans lequel les systèmes de connexion (10) sont organisés selon un arrangement matriciel.

13. Système ultrasonore selon la revendication 11 comprenant une unité de connexion comprenant :
- un premier membre (11a₁) appartenant au premier ensemble et un premier membre (11b₁) appartenant au deuxième ensemble,
- un seul dispositif d'activation (14), et
- un seul troisième membre (13) déplacé par ledit dispositif d'activation et adapté pour recevoir et verrouiller un deuxième membre (12₁) d'une première sonde (3₁) pour établir des contacts électriques entre ledit deuxième membre (12₁) et le premier membre (11a₁) appartenant au premier ensemble, et adapté pour recevoir et verrouiller un deuxième membre (12₂) d'une deuxième sonde (3₂) pour établir des contacts électriques entre ledit un deuxième membre (12₂) et le premier membre (11b₁) appartenant au deuxième ensemble.
